# EUROPEAN PATENT APPLICATION

(11) **EP 2 402 433 A1**
(43) Date of publication of application: **04.01.2012**
(21) Application number: 10168159.1
(22) Date of filing: 01.07.2010
(51) Int. Cl.: C12N 1/38

(54) **Method for increasing the expression of a recombinant protein and plasmid yield in an eukaryotic or prokaryotic high cell density culture**

(71) Applicant: BioSilta Oy, 90014 Oulu (FI)
(72) Inventor: Pilarek, Maciej, 88 100 Inowroclaw (PL); Neubauer, Peter, 13467 Berlin (DE)
(74) Representative: Ganahl, Bernhard

(57) **Abstract**

The present invention relates to a method for increasing the expression of a recombinant protein and plasmid yield in an eukaryotic or prokaryotic high cell density culture. The increase of the expression is achieved by the use of perfluorochemicals as ingredients of an enzyme-based fed-batch cultivation system by enhancing the cell growth rate and the final cell density.

## Description

The present invention relates to a method for increasing the expression of a recombinant protein and plasmid yield in an eukaryotic or prokaryotic high cell density culture. The increase of the expression is achieved by the use of perfluorochemicals as ingredients of an enzyme-based fed-batch cultivation system by enhancing the cell growth rate and the final cell density.

### BACKGROUND OF THE INVENTION

The vast majority of biopharmaceuticals to date are therapeutic protein drugs, i.e. recombinant proteins, which are engineered in the laboratory. Genetic engineering techniques were discovered first by Cohen et al. (1973), Proc. Natl. Acad. Sci. U.S.A. 70, 3240-3244, who could "cut and paste" a segment of a DNA and reproduce (clone) the new DNA in bacteria. Up to now, these early techniques have further been developed to a technology of producing proteins in foreign hosts. The technology of recombinant gene expression enables the production of high value proteins (e.g. for therapeutic use), which are often limited by their low availability. In the meantime recombinant proteins have been approved for therapeutic use and are industrially produced. A whole industry around this technology has been developed.

Today, recombinant proteins are used to treat patients suffering from many conditions, including various cancers, heart attacks, strokes, cystic fibrosis, Gaucher's disease, diabetes, anaemia and haemophilia. The leading classes of recombinant proteins are erythropietins, monoclonal antibodies and interferons, whereas specific examples of recombinant proteins are human insulin, somatropin, recombinant tissue-type plasminogen activator, hepatitis B antigen, epoetin alpha and epoetin beta (Zündorf, I. and Dingermann, T., 2000, Pharmazie in unserer Zeit 29, 167-173).

Different host systems are available for the production of recombinant proteins: eukaryotic cells, such as cells from yeasts, filamentous fungi, insects, plants, mammals, and prokaryotes including different bacteria, such as *Escherichia, Bacillus* and *Staphylococcus.* The production of recombinant proteins in microbial cells represents the most convenient and cost-effective method. *Escherichia coli* has become the most extensively used bacterial host since it is a genetically and physiologically well-characterised organism that grows fast and requires simple media. General problems during the production of recombinant proteins in *E. coli,* however, are the culture growth rate and the final cell density, which are directly proportional to the expression amount of the recombinant protein to be produced.

Aside from protein production E. coli is also used as a host for plasmid production. Plasmid DNA is mainly used as vaccines and in gene theraphy. The yield of plasmids depends on plasmid factors (replication origin) but also on the cultivation procedure.

During a growth process of respirating organisms, including cell cultures and bacteria, which is carried out in media providing sugars, such as glucose, as a carbon and energy source, respiratory activity, sugar consumption and the growth or the bacterium are closely related.

High cell densities, which are a basis for high volumetric yield of the product, i.e. the recombinant protein, are not obtained usually in a so called "batch process" which contains all substrate from the beginning due to substrate inhibition and unbalanced growth (accumulation of toxic side metabolites, exponential increase of all cellular reactions leading to oxygen limitation), but dense cultures can be obtained by continuous feeding of a concentrated sugar solution or other carbon source to a culture in a bioreactor. A process performed in this way is called a "fed-batch process", and importantly the feed rate of the carbon source can be adapted for a good performance rate of the process, i.e. control of the cellular reactions for maximum biomass and product amount or product quality. The feeding of the carbon source is generally performed by pumping the solution into a bioreactor, however also internal supply strategies have been recently described. An example are the EnBase^{®} and EnBase Flo streategies which relies on enzymatic processing of a polymeric substrate, consisting of a straight or branched chain of monomers of the natural carbon source (EP 09154440.3, EP 08 157 594.6, PCT/EP2009/056861; PCT application PCT/F12007/050648),. This polymeric substance is degraded by an enzyme which is added to the cultivation medium and the rate of substrate supply is controlled by the amount of the enzyme.

The cell density to be obtained in a cultivation medium is limited by the oxygen transfer, as the oxygen solubility in water solutions is very low and correspondingly this oxygen amount is consumed in a very short time. Conseqeuntly there is a need of continuous oxygen supply which is generally obtained by shaking the cultures or sparging the culture browth with air or oxygen enriched air. The maximum cell density of cell cultures is limited by the efficiency of the oxygen transfer. If oxygen supply is limiting the growth the cell yield on the sugar becomes very low due to the formation of organic fermentation products (acetate, formic acid, lactic acid, ethanol, succinic acid) in quantities. Additionally, these organic molecules inhibit the growth of bacteria and impair recombinant protein production.

Thus, the problem of the present invention is how to increase the oxygen transfer capacity in cultures of eukaryotic or prokaryotic cells, which are cultivated in a fed-batch system in order to enhance the culture growth rate, the final cell density, and plasmid and protein production.

This problem is solved by a method as defined in independent claim 1. Preferred embodiments are the subject-matter of the dependent claims.

### SUMMARY OF THE INVENTION

The present invention relates to a method for increasing the expression of a recombinant protein or the yield of a plasmid in a eukaryotic or prokaryotic cell culture cultivated in an enzyme-based fed-batch cultivation system using a cultivation medium which is supplemented with at least one perfluorochemical.

The increase of the expression of a recombinant protein is realized by controlling the growth rate and the final cell density of the eukaryotic or prokaryotic cells. In a preferred embodiment the cultivation system can be further improved by the addition of an amylolytic enzyme and a boosting solution containing highly concentrated amino acids and peptides.

In particular, by the supplementation of the cultivation medium with perfluorochemical(s) an increase of the oxygen transfer capacity is achieved. Thus, in an enzyme-based fed-batch system where the growth-limiting substrates are provided by enzymatic release more enzyme can be added and, consequently, the culture growth rate and final cell density can be increased, which may influence the expression of the recombinant protein or plasmid to be produced.

### BRIEF DESCRPTION OF THE DRAWINGS

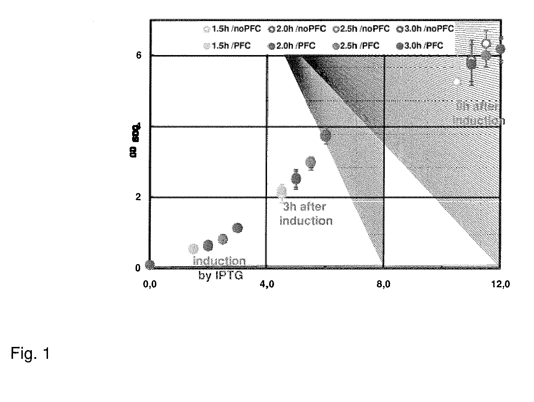

Figure 1 shows the growth of E. *coli* RB791 pQE30adh cells in 24 deepwell plates on LB-medium at 30°C with induction of ADH at different cell densities. Half of the cultures was supplemented with PFD_{40%} (marked as "PFC"). The other half of the cultures was not supplemented with any PFD (marked as "no PFC") as a control. Cultured cells were inducted by IPTG (1 mM) at various time points (at 1.5, 2.0, 2.5 and 3.0 h). OD₆₀₀ values have been measured at the time of induction as well as at 3 and 9 hours after induction (as it was marked on diagram). The initial OD₆₀₀ of all cultures was 0.1.

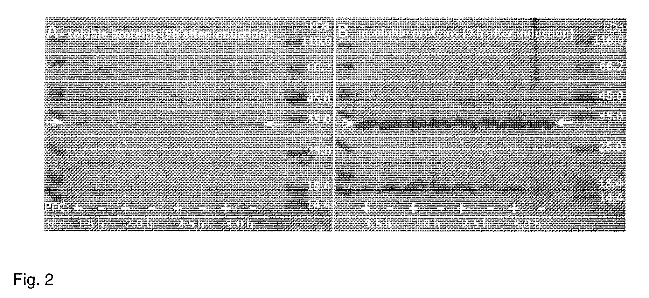

Figure 2 shows the SDS-PAGE analysis of soluble (A) and insoluble (B) protein fragments obtained from *E. coli* RB791 pQE30adh cells cultured on LB-medium. Half of the cultures was supplemented with PFD_{40%} (marked as "+"). The other half of the cultures was not supplemented with any PFC (marked as "-") as a control. Cultured cells were inducted by IPTG (1 mM) at various time points (at 1.5, 2.0, 2.5 and 3.0 h; marked as "ti"). The arrows show the position of the ADH-bands.

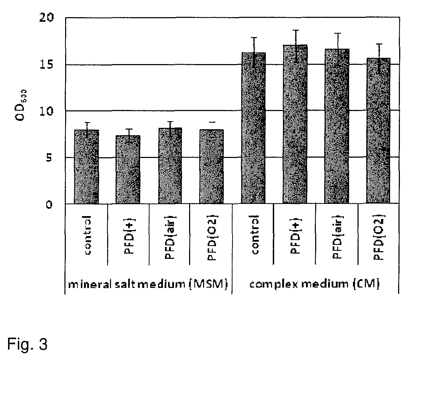

Figure 3 shows OD₆₀₀ values measured after 12 hours for E. *coli* RB791 pQE30adh cells cultured in EnBase^{®}-Flo Mineral Salt Medium (MSM) and EnBase^{®}-Flo Complex Medium (CM) after supplementation with unsaturated perfluorodecalin (PFD₊) and perfluorodecalin saturated by compressed air (PFDₐᵢᵣ) or pure oxygen (PFD_{O2}). Values marked as "contro!" were measured for the control cultures, which were not supplemented with any PFC.

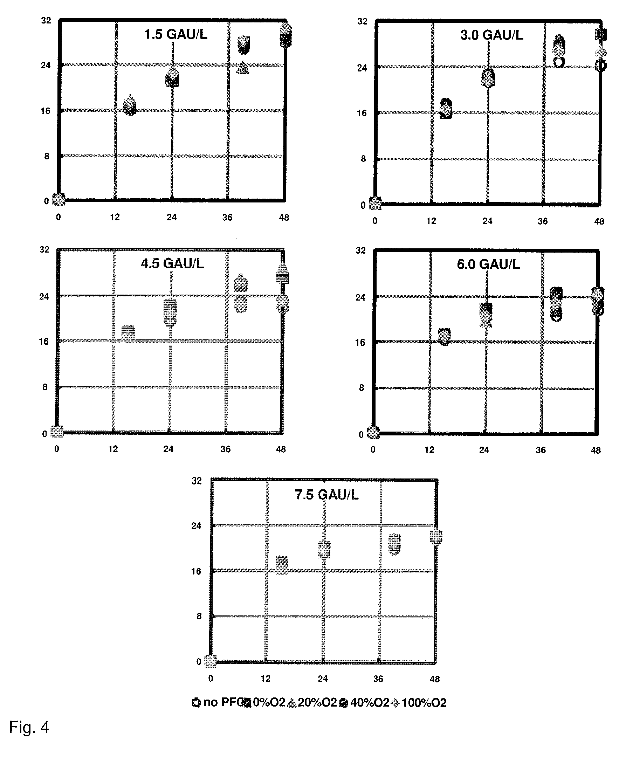

Figure 4 shows the OD₆₀₀ values measured for E. *coli* RB791 pQE30adh cells cultured in EnBase^{®}-Flo Complex Medium (CM) after supplementation with PFD saturated by compressed air (PFD_{20%}), or further enhancing the oxygen level to 40% (PFD_{40%}) or saturating PFD by pure oxygen (PFD_{100%}). Values marked as "no PFC" were measured for the control cultures, which were not supplemented with any PFC; PFD_{0%} is a control where PFD was not treated with either rair or oxygen. Final concentrations of the amylolytic enzyme amyloglucosidase (GAU) after 15 hours of culturing: 1.5; 3.0; 4,5; 6.0 and 7.5 GAU L⁻¹.

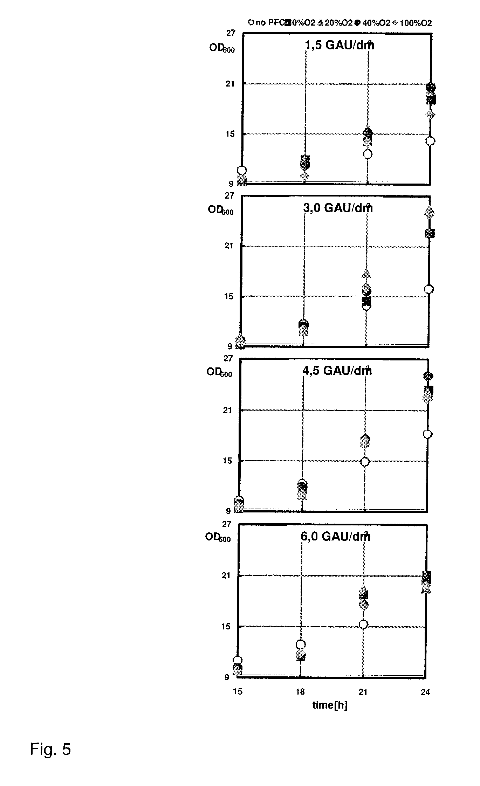

Figure 5 shows the OD₆₀₀ values measured for E. *coli* RB791 pQE30adh cells cultured in EnBase^{®}-Flo Mineral Salt Medium (MSM) after supplementation with PFD saturated by compressed air (0% extra O₂), PFD saturated by oxygen enriched air (20% extra O₂, 40% extra 0₂) and PFD saturated by pure oxygen (100% pure O₂). Values marked as "no PFC" were measured for the control cultures, which were not supplemented with any PFC. Final concentrations of the amylolytic enzyme amyloglucosidase (GAU) after 15 hours of cultivation were 1.5, 3.0, 4.5 and 6.0 GAU L⁻¹.

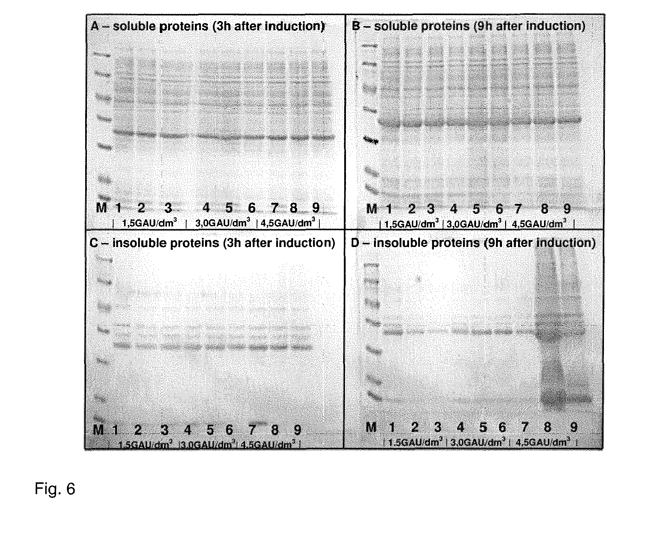

Figure 6 shows the comparison of the SDS-PAGE analysis of soluble (A,B) and insoluble (C,D) heterologous alcohol dehydrogenase (ADH) expressed in E. *coli* RB791 pQE30adh cells cultured in EnBase^{®} MSM medium supplemented with PFD saturated by compressed air (PFD_{0%}), PFD saturated by air enriched with 40% oxygen (PFD_{40%}) and control cultures, which where not supplemented with any PFC (no PFC): M - molecular weight marker; 1,4,7 - cells cultured without PFD (no PFD); 2,5,8 - cells cultured with PFD_{0%}; 3,6,9 - cells cultured with PFD_{40%}. Final concentrations of the amylolytic enzyme amyloglucosidase (GAU) were 1.5, 3.0 and 4.5 GAU L⁻¹.

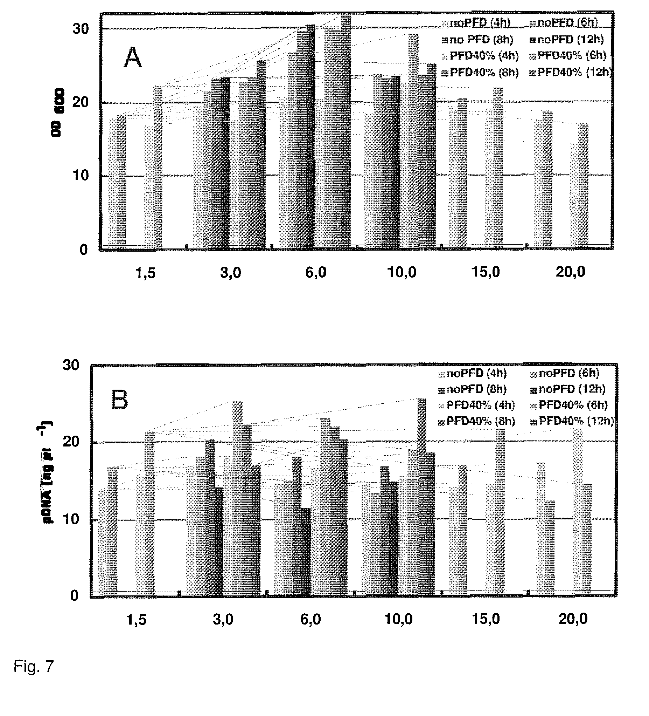

Figure 7 shows the OD₆₀₀ values (A) and values of the plasmid concentration (B; pDNA; ng µL⁻¹) measured for E. *coli* Top 10 cells cultured in EnBase^{®}-Flo Complex Medium (CM) after supplementation with PFD saturated by air enriched with 40% oxygen (PFD_{40%}) after 4, 6, 8, and 12 h of cultivation. Values marked as "no PFD" were measured for the control cultures, which were not supplemented with any PFC. Final concentrations of the amylolytic enzyme amyloglucosidase (GAU) after 15 hours of cultivation were 1.5, 3.0, 6.0, 10.0, 15.0 and 20.0 GAU L⁻¹.

### DEFINITIONS

As used herein, "microbial" or "microbe", refers to organisms, also known as "microorganisms", that are microscopic, i.e. usually too small to be seen by the naked human eye.

As used herein, "prokaryotic cells" or "microbial prokaryotic cells", also known as "prokaryotes", refer to a group of mostly unicellular organisms that lack a nucleus, also known as karyon, or any other membrane-bound organelles. The prokaryotes are divided into two domains, i.e. archaea (archaebacteria) and bacteria (eubacteria). Preferred prokaryotic cells are *Escherichia coli, Bacillus subtilis, Bacillus licheniformis, Bacillus megaterium, Lactobacillus lactis, Streptomyces lividans.*

As used herein, "eukaryotic cells", also known as "eukaryotes", refer to organisms, whose cells are organized into complex structures enclosed within membranes, e.g. the nucleus. Preferred eukaryotic cells are yeasts, such as *Saccharomyces cerevisiae* and *Pichia pastoris,* or filamentous fungi, such as *Trichoderma reesei* and *Aspergillus niger,* or higher cell cultures such as insect, hamster cells (BHK21, CHO) or human cell lines. Yeasts and filamentous fungi are also used herein as belonging to the group of microbes.

As used herein, "cell culture" or "cell cultivation" is a method of multiplying microbial organisms, i.e. by which microbial prokaryotic or eukaryotic cells are grown under controlled laboratory conditions.

As used herein, "plasmid" refers to an extrachromosomal selfreplicating DNA element which contains additional genetic information, such as the gene(s) for the product, at least one replication origin and a selection marker which can be an antibiotic resistance or a gene which complements a function of a host cell. Plasmids ar mostly circular, but also linear plasmids are known. Examples of plasmids are derivatives of pBR322 having the ColE1 replication origin, such as the pUC and pET plasmids. Other examples contain the replication origins of p15A, such as the pACYC184 plasmid, or the origin of the low copy plasmids F', P1 or R.

As used herein, "high cell density" refers to a cultivation which yields a high number of microbial prokaryotic or eukaryotic cells in a defined period of cultivation. The high-cell-density value is dependent on the cell type and species and can be defined as the cell density value that is reached with gradual addition of growth-limiting substrates (fed-batch) without intoxicating the cell culture, i.e. a viable cell concentration of 5x10⁹ cells/ml, preferably above 1x10¹⁰ cells ml⁻¹ of *E. coli,* or a cell concentration above 1x10⁷ for other cell cultures

As used herein, "laboratory-scale" liquid cultures refer to cultivation of microbial prokaryotic or eukaryotic cells in bioreactors or the like in the range of 500 ml to 10 l, shake flasks or the like in the range of 10 to 1000 ml, in cuvettes, glass vials, falcon tubes or the like in the range of 1 to 100 ml, in microtiter plates, microbioreactors or the like in the range of 0.001 ml(10 µl) to 10 ml.

As used herein, "large-scale" liquid cultures refer to cultivation of microbial prokaryotic or eukaryotic cells in cultivation vessels from 10 liters up to 1500 m³, preferably 100 liters to 100 m³, more preferably 100 to 1000 liters. They are typically performed in bioreactors (e.g. stirred tanks), but can also include simple (disposable) containers like plastic bags of up to few cubic meters.

As used herein, the cultures are performed in any suitable formate, e.g. eppendorf tubes or other small vials, on multiwell plates or other platforms with many small culture holes which may be shaken or otherwise mixed; application in/on plates of automatic cultivation units (robots); mikrosegmented cultivation tubes (e.g. from HKI, Jena, Germany). In a preferred embodiment the cultures are shaken during the cultivation process. The shaking volocity is from 10 to 1500 rpm, preferably from 80 to 1200 rpm and depends from the amplitude of the shaker which varies from 1 mm to 5 cm. Generally for obtaining a good oxygen transfer, if the amplitude of the shaker is smaller, the shaking speed must be increased (e.g. amplitude of 2.5 cm and 350 rpm or 1 mm amplitude and 1000 rpm for 96 well plates)(see Hermann et al., 2003, Biotechnol. Bioeng. 81 (2), 178-186). Often the amplitude is larger and the shaking speed lower for larger cultures, e.g. shake flasks (e.g. 5 cm amplitude and 250 rpm for 1000 ml shake flasks).

As used herein, "batch" cultivation is a discontinuous process, where the sterile growth medium with all substrates required is initially inoculated with a pure culture of microbial prokaryotic or eukaryotic cells and no additional growth medium is added during the course of operation. This means, that the batch process is a partially closed system, wherein the only material added and removed during the course of operation is air/gas exchange, antifoam and pH controlling agents. The batch cultures are continuously shaken or stirred to keep a desired degree of homogeneity of the substrates and cells and to guarantee an as high as possible oxygen transfer for aerobic cultures.

As used herein, "fed-batch" cultivation is a process, where a certain amount of fresh growth-limiting substrates is continuously added to the cultivation medium to provide their low concentrations in cultivation media and to obtain control of the growth of microbial prokaryotic or eukaryotic cells. The "enzyme-based fed-batch technique" (preferably "EnBase^{®}-Flo" system; Krause et al., 2010. High cell-density cultivation method from BioSilta challenges traditional shake flask cultivation systems: Fed-batch based cultivation system gives improved yield of high quality protein. Microb Cell Fact. 9:11) refers to a technique by which the growth-limiting substrate-monomer is slowly released from a substrate-polymer or substrate-oligomer either enzymatic degradation from a solid phase or by enzymatic degradation from a water soluble or partly soluble substrate-polymer or substrate-oligomer containing in the liquid phase. In this case the substrate-polymer or substrate-oligomer can be added continuously or discontinuously to the culture, but referentially is added at the beginning of the culture. In this regard it is also possible to add one or more portions of the substrate-polymer or substrate-oligomer, of the substrate-polymer or substrate-oligomer degrading enzyme during the fermentation and "boost" with nitrogen compounds depending on the growth.

As used herein, the "substrate" refers to any suitable substance, which affects the cultivated organisms' capability to grow.

As used herein, the "substrate-monomer" refers to a metabolically active growth component, which can be efficiently degraded by the microbial prokaryotic or eukaryotic cells of the culture. In the present invention the substrate-monomer is preferably "growth-limiting", which means that the substrate-monomer is present in a low proportion in relation to the other ingredients and that it is exhausted first. En example for a growth-limiting substrate monomer is glucose.

As used herein, the "substrate-polymer" or "substrate-oligomer" refers to a metabolically inactive growth component, which cannot be efficiently degraded by the microbial prokaryotic or eukaryotic cells of the culture. Preferably the "substrate-polymer" or "substrate-oligomer" has minimum length of two monomers. Preferably the "substrate-polymer" or "substrate-oligomer" is water-soluble.

As used herein, the "enzymes" refer to all enzymes known in the art capable of degrading the substrate-polymer or substrate-oligomer for the release of the growth-limiting substrate-monomer to provide fed-batch conditions. For enzymatic degradation of the substrate-polymer or substrate-oligomer, either individual enzymes or combinations of different enzymes, i.e. enzyme cocktails can be applied. Preferred enzymes are amylolytic enzymes.

As used herein, the "glucose polymer" refers to homopolymers or heteropolymers. The "homopolymer" refers to a glucose polymer, which consists of D-glucose monomers linked by glycosidic bonds and the "heteropolymer" refers to a compound comprised of different monosaccharides or aminosugars, e.g. hemicellulose.

As mentioned herein, EnBase is a miniaturized fed-batch cultivation system for high cell density cultivation of microorganisms (Panula-Perala, J. et al., 2008, Micorbial Cell Factories 7: 31; BioSilta Oy, Oulu, Finland). In this system, glucose is controllably supplied by the use of an internally dissolved glucose polymer, which cannot be directly assimilated by growing cells, and by the application of an amylolytic enzyme, which releases single glucose monomers. from substrate-polymers or substrate-oligomers. Since the amount of the enzyme determines the release rate of glucose, such fed-batch cultivation allows the control of the culture growth rate and the final cell density. Thus, such fed-batch cultivation provided by glucose supplementation of the culture medium with the internal enzymatic reaction allows the regulated control of microbial cell growth. As mentioned herein, EnBase^{®} Flo is a variant of EnBase^{®} in which the polymer is fully dissolved in the growth medium and is not stored in a solid or gel phase.

As used herein, PFD_{x%} is a perfluorodecalin solution which contains a defined oxygen content. Therefore as a starting material two PFD solutions were prepared; (i) was saturated with air, and (ii) was saturated with pure oxygen by bubbling the respective gasses through the solutions. Air saturated PFD contains 1.02 mmol/I of oxygen and oxygen saturated PFD contains 36 mmol/I oxygen (both at 37 °C). PFD_{X}, is a mixture which contains x% of the oxygen saturated PFD and (100-x)% of air saturated PFD. As an example PFD_{40%} is a mixture of 40% of oxagen saturated PFD and 60% of air saturated PFD. Correspondingly PFD_{0%} is a solution with only contains air saturated PFD.

### DETAILED DESCRIPTION OF THE INVENTION

The present invention relates to a method for increasing the expression of a recombinant protein or production of a plasmid in a eukaryotic or prokaryotic cell culture cultivated in an enzyme-based fed-batch cultivation system using a cultivation medium which is supplemented with at least one perfluorochemical.

"At least one perfluorochemical" means one or several (2, 3, 4, 5, 6 or more) different perfluorochemicals.

There are different kinds of oxygen vectors, which can be used as oxygen carriers in the biotechnology, such as modified hemoglobin derivatives, siloxylated copolymers, silicone oils or hydrocarbons. However, the use of perfluorchemicals (PFC) as oxygen carriers has some advantages in comparison with the use of other kinds of oxygen carriers. For example, an important advantage is their lack of toxicity and the absence of negative side effects on various kinds of living cells. These advantages have been confirmed by experimental results and clinical researches (Mattiasson, B. and P. Adlercreutz, 1987, Trends in Biotechnology, 5: 250-254; Krafft, M.P., 2001, Advanced Drug Delivery Reviews, 47: 209-228; Lowe, K.C., 2002, Journal of Fluorine Chemistry, 118: 19-26; Riess, J.G., 2006, Artificial Cells, Blood Substitutes and Biotechnolgy, 34: 567-580). Furthermore, it is advantageous that PFCs are non-miscible with aqueous media and that they create a separate phase (known as perfluorinated phase) below, i.e. on the bottom, of the aqueous phase. Thus, the volume of the PFCs added to the culture medium does not change the concentration of the other medium components. As they are non-miscible with the water phase, PFCs can be also effectively recovered from the culture system and then reused. Another merit of PFCs is the possibility to sterilize them by heat (e.g. by autoclaving) and their chemical stability, which makes them easy to store, e.g. at room temperature.

Perfluorochemicals (PFC), also known as fluorocarbons or perfluorocarbons, are synthetic fluorine-substituted derivatives of hydrocarbons, i.e. they are similar to hydrocarbons but several, if not even a majority or all, hydrogens are replaced by fluoride. Due to the strength of the carbonfluorid, they are stable and inert compounds with a high resistance to heat (Mattiasson, B. and P. Adlercreutz, 1987, Trends in Biotechnology, 5: 250-254; Lowe, K.C., 2002, Journal of Fluorine Chemistry, 118: 19-26; Riess, J.G., 2006, Artificial Cells, Blood Substitutes and Biotechnolgy, 34: 567-580).

Liquid PFCs are characterized by a high solubility of oxygen, carbon dioxide and other non-polar gases, which have gained much interest in medical and technical applications (Krafft, M.P. et al., 2001, Advanced Drug Delivery Reviews, 47: 209-228; Lowe K.C., 2001, Journal of Fluorine Chemistry, 109: 59-65; Riess, J.G., 2006, Artificial Cells, Blood Substitutes and Biotechnolgy, 34: 567-580). For example, oxygen solubility in PFC liquids used for biomedical applications is typically 35-44 mmol L⁻¹, as compared to 2.2 mmol L⁻¹ for water. The solubility of oxygen in PFCs is inversely proportional to the molecular weight and directly proportional to the number of fluorine atoms present. In contrast to the characteristic sigmoid relationship between the oxygen content and the partial pressure (dissociation curve) for the natural respiratory pigment, haemoglobin, the gas solubility in PFCs increase linearly with the partial pressure approximating to Henry's Law. Gas molecules are believed to occupy so-called 'molecular cavites' within liquid PFCs, but no chemical reactions are involved. Consequently, for oxygen, the gas release from a PFC liquid occurs more rapidly than the dissociation of oxyhaemoglobin (King, A.T. et al., 1989, Biotechnology, 7: 1037-1042; Lowe, K.C., 2002, Journal of Fluorine Chemistry 118: 19-26; Riess, J.G., 2006, Artificial Cells, Blood Substitutes and Biotechnolgy, 34: 567-580).

Such unique properties make the PFCs suitable for the development of culture media with a facilitiated oxygen transport and, thus, lead to an enhanced growth rate and final cell density in cultures of various kinds of cells.

Thus, the present invention provides a cell culture method, wherein eukaryotic or prokaryotic cells carrying a recombinant vector are grown in an enzyme-based fed-batch cultivation system to a high cell density wherein to the cultivation medium a perfluorochemical is added. The present invention makes the recombinant expression much more independent from the time of induction and, thus, is much more robust than any known recombinant expression.

Preferably, the cell culture method is used for the cultivation of bacterial cells, more preferably for the cultivation of *Escherichia coli* cells. The most prefered E. *coli* strains are E. *coli* RB791 and E. *coli* Top 10 expressing any type of a recombinant protein. One exemplary recombinant protein is the heterologous alcohol dehydrogenase (ADH) contained in a plasmid named pQE30adh.

For plasmid production the most preferred E. *coli* strains are *E. coli* TOP10*,* E. *coli* DH5□ and E. *coli* DH10□. The most preferred plasmids contain a ColE origin of replication but other origins of replication may also be used.

Moreover, the EnBase^{®}-Flo system as defined above is the most prefered enzyme-based fed-batch cultivation system to which a perfluorochemical is added.

As perfluorochemicals (PFCs) liquid perfluorochemicals are the most preferred perfluorinated oxygen carriers. More preferably, perfluordecalin (PFD; C₁₀F₁₈; 1,1,2,2,3,3,4,4a,5,5,6,6,7,7,8,8a-octadecafluorodecalin) is identified as a suitable liquid perfluorochemical. Different types of PFDs are possible:
- PFD saturated by compressed air (PFD_{0%}),
- PFDs with a higher concentration of oxygen, which are prepared by mixing different amounts of PFD saturated by air with oxygen enriched PFD solutions. Thus PFD solutions were prepared which contained 20, 40, 60, or 80% of oxygen saturated PFD (PFD_{20%}, PFD_{40%} PFD_{60%}, PFD_{80%}),
- PFD saturated by pure oxygen (PFD_{100%}).

Moreover, amylolytic enzymes are the most preferred enzymes, which are necessary for the degradation of the substrate-polymer or substrate-monomer during the miniaturized fed-batch cultivation system. The enzyme amyloglucosidase (GAU) represents an exemplary degrading enzyme.

The miniaturized fed-batch cultivation system can be performed with any type of medium, preferably mineral salt media (MSM) which may additionally contain a complex component. The preferred complex media are LB-medium, superbrowth and Terrific broth. Preferred special media are MSM- and CM-Medium from Biosilta Oy, Oulu, Finland (EnBase^{®}-Flo media), wherein MSM-medium is the most preferred.

In the first preferred embodiment, the miniaturized fed-batch cultivation system (EnBasee^{®}-Flo) is performed with MSM-medium, either supplemented with an initial amount of the amylolytic enzyme, or the amylolytic enzyme is added after an adaptation phase of 1-3 hours. Furthermore, the MSM-medium is supplemented with a perfluorinated oxygen carrier, such as PFD saturated by air enriched with pure oxygen (aerated/oxygenated), more preferably aerated/oxygenated PFD, wherein the PFD_{40%}) mixture , containing 40% of oxygen enriched PFD and 60% of air enriched PFD) is the most preferred perfluorinated oxygen carrier. Preferably, the aerated/oxygenated PFCs are added at the time of inoculation, but most preferably at the time, when the cells are growing at a high cell density, e.g. 15 hours after inculation.

In a second preferred embodiment, the above mentioned media, which are already supplemented with the initial amount of the amylolytic enzyme and the aerated/oxygenated PFD, are additionally supplemented with further amounts of the amylolytic enzyme, such as further amounts of the enzyme glucoamylase (GA), in order to obtain final concentrations of about 1.5 - 20 units L⁻¹ more preferably 1.5, 3.0, 4.5, 6.0, 7.5, 10.0, 15.0 or 20.0 units L⁻¹. The lower final concentrations of 1.5 - 4.5 units L⁻¹ are more preferred because, otherwise, the final concentrations of the substrate-monomer may be too high. The final concentration of 4.5 units L⁻¹ is the most preferred concentration of the amylolytic enzyme. These further amounts of the amylolytic enzyme are preferably added, when the cells are growing at a high cell density, e.g. 15 hours after inoculation.

In a third preferred embodiment, the above mentioned media, which are already supplemented with the initial and the additional amount of the amylolytic enzyme and with the aerated/oxygenated PFD, are additionally supplemented with complex components, such as a mixture of tryptone and yeast extract. The complex components are preferably added at the time of the inoculation but most preferably at the time, when the cells are growing at a high cell density, e.g. 15 hours after inculation.

In the most preferred embodiment, the *E. coli* cells are cultivated in a phosphate buffered mineral salt medium, such as in BioSilta Oy's MSM-Medium in the EnBasee^{®}-Flo or EnPresso^{TM} miniaturized fed-batch cultivation systems, wherein the final concentration of the substrate-polymer is preferably between 10 and 50 g L⁻¹ and the concentratrion of the polymer- degrading enzyme is 4.5 units L⁻¹, which is obtained by an initial and a further addition of the amylolytic enzyme and wherein the medium is supplemented with PFD_{40%} and a boosting solution providing a final concentration of 12 g L⁻¹ tryptone and 24 g L⁻¹ yeast extract at the time, when the cells are growing at a high cell density.

The best possible yield of the expression of the recombinant protein is achieved, when the perfluorochemicals, the additional amount of the amylolytic enzyme and the boosting solution are added, when the bacterial cells grow at a high cell density, for example 15 hours after inoculation.

The invention is further described in more detail in the following examples which are not construed as limiting the scope of the invention.

### EXAMPLES

### Example 1. Effects of the cell culture supplementation with air/O2 saturated PFD on the growth rate and the time of induction of low cell density E. coli RB791 pQE30adh cultures (cultivated in LB-medium)

### a) Bacterial strains

The recombinant strain of *Escherichia coli* RB791 pQE30adh expressing the heterologous alcohol dehydrogenase (ADH) was used (see Viitanen et al. 2003. Microbial Cell Factories 2, 1-15.).

### b) Media composition

The medium used was LB-medium (10 g/I trypton, 5 g/I yeast exctract, 5 g/I NaCl, pH was set as 7.0).

### c) Perfluorinated oxygen carrier

Perfluorodecalin (PFD; C₁₀F₁₈ 1,1,2,2,3,3,4,4a,5,5,6,6,7,7,8,8,8a-octadecafluorode-calin; ABCR GmbH & Co. KG, Karlsruhe, Germany) was used as the liquid perfluorinated oxygen carrier. The perfluorochemical was sterilized by autoclaving and then cooled to room temperature. Sterilized and cooled perfluoroalkane was saturated by compressed air and pure oxygen in aseptic conditions supplied by 0.2 µm cartridge filter (Pilarek, M. and K.W. Szewczyk, 2008, Biochemical Engineering Journal, 41: 38-42). The oxygen carrier in these experiments was PFD_{40%}, a 60:40 mixture of air saturated PFD air and oxygen saturated PFD.

Also unsaturated perfluorodecalin (PFD₍₊₎) was used in the experiments to check possible side-effects triggered off by the perfluorochemical. The culture, which was not supplemented with perfluorodecalin (no PFD), was used as reference. The perfluorinated oxygen carrier has created a separate phase on the bottom of the wells after pipeting as it cannot mix with aqueous systems. Thus, the perfluorodecalin volume, which was added to the culture, does not change the concentration of the ingredients of the cultivation medium.

### d) Analytical methods

The growth of the cells was monitored by spectrophotometrical measurement of the light absorbance of the cell suspension samples collected during culturing. The analytical length of the OD₆₀₀ method was λmax = 600 nm. The OD₆₀₀ value of 1 is equivalent to 1.7 g L⁻¹ of wet weight and to 0.39 g L⁻¹ of dry weight of E. *coli* RB791 pQE30adh biomass.

The soluble and the insoluble fraction of cellular proteins was analyzed by the SDS-PAGE method. Cells' suspension samples were standardized to OD₆₀₀ = 10 and the cells were centrifuged (13 000 rpm for 3 min. in 4°C) to separate the cells from the medium culture. The pellet was resuspended in 1.0 ml of 0.1 M Tris/HCI (1.0 mM EDTA, pH 7.0) buffer and 2.0 µl of lysozyme (50 mg/ml) was added. The suspension of the cells was incubated with lysozyme for 30 minutes at 0-4°C. After incubation, the sample was sonicated (3 x 30 sec with 45 sec off, amplitude 70%) at 0-4°C. The centrifugation (13 000 rpm for 3 min in 4°C) lets separate fractions of soluble (in supernatant) and insoluble (in pellet) proteins. The supernatant was directly used as the soluble protein sample. Pellets of the insoluble protein fraction were resuspended again in 1.0 ml of 0.1 M Tris/HCI (1.0 mM EDTA, pH 7.0) buffer before analyzing. Samples were separated in 12% polyacrylamid gels and two steps of electrophoresis were done: (1) 60 V for 30 min and then (2) 90 V for 90 min. After the separation, the gels were stained with Coomassie Brillant Blue R 250 solution according to standard staining method modified by Besir (Besir, H., 2008, Lab Times, 6: 53).

### e) Cultivation

An E. *coli* RB791 pQE30adh inoculum was prepared from a pure culture cultivated overnight on a Petri dish with LB-medium at 30°C. The preculture was washed with 3.0 ml of fresh EnBase^{®}-Flo-medium and after OD₆₀₀ measurement the suspension of precultured cells was used to inoculate cultures with initial cultivation OD₆₀₀ = 0.15.

Cultivations of E. *coli* RB791 pQE30adh cells were performed in 24-well plates. Initially all cultures were done in 3.0 ml of the medium. Multiwell plates were covered by gas-permeable membrane (without additional sealing) to provide aeration. Cultures were incubated at 30°C on orbital shakers (250 rpm).

Some of the cultures were supplemted with 3.0 ml gas saturated PFD.

All cultures were supplemented with 30 µl of a 100 mM solution of isopropyl β-D-1-thiogalactopyranoside (IPTG; Carl Roth GmbH, Karlsruhe, Germany) to obtain 1.0 mM concentration of the plasmid inducer in the cultures.

100 µl samples of cells suspended in culture medium were harvested during the experiments in order to analyze the OD₆₀₀, and for the SDS-PAGE separation of cellular proteins. The average rate of water evaporation from EnBase^{®}-Flo medium through air permeable membrane was calculated as 9.1 µl h⁻¹ for every used well of the multiwell plate.

### f) Results and Discussion

Firstly, it has been investigated whether the supplementation with PFD has an effect on the growth and the production of ADH in deepwell plate cultures. Therefore, *E. coli* RB791 pQE30adh cells were grown in complex medium (LB) in deepwell plates at 30 °C. Half of the cultures were cultivated with a bottom phase of PFD, which was saturated by 40% oxygen enriched air (PFD_{40%}); the other cultures were performed without PFD (control). All wells of the plate were inoculated at the same time but were induced at four different time points (1.5, 2.0, 2.5 and 3.0 h), where different cell densities between OD₆₀₀ 0.4 and 1.2 had been measured. Although we could not detect any negative effects of PFD on the development of the cell density, i.e. on the growth rate, also no positive effects could be observed. As it turned out, the use of a perfluorinated oxygen carrier cannot improve the growth of *E*. *coli* cells cultured on complex medium at relatively low cell densities (see Fig. 1).

Furthermore, ADH accumulation in the soluble or insoluble protein fractions was analyzed by SDS-PAGE at the end of each experiment (9 hours after induction). Generally, high expression of ADH could be observed in all analyzed samples (see Figs. 2A and B). The product accumulated in the soluble fraction (see Fig. 2A) and as inclusion bodies, i.e. in the insoluble fraction (see Fig. 2B). Whereas the amount of inclusion bodies was the same in all samples (see Fig. 2B), clear differences could be observed for the soluble protein (see Fig. 2A). Without PFDs, the highest amount of soluble ADH was seen in the culture, which was induced at the lowest cell density (after 1.5 h), whereas the induction at higher cell densities (after 2.0-3.0 h) resulted in a drastic reduction of the soluble product. This is generally not surprising and widely known, that the cell density of induction in shaken cultures is a very critical parameter for the production of the recombinant protein. Usually, slight changes in the time of induction, i.e. at different cell densites, have big effects. Therefore, it was very surprising that in the case of added air/O₂ saturated PFD, good accumulation of ADH was obtained in all cultures, i.e. the time of induction was not a critical parameter any more. These initial experiments suggest that, if the shaken cultures are supplied with gas saturated PFD, the recombinant expression is much more independent on the induction time and, thus, much more robust.

This example shows that the supplementation with air/O₂ saturated PFD has no effect on the growth rate of low cell density E. *coli* RB791 pQE30adh cultures, but ensures that the success of recombinant protein production is more robust, i.e more independent, from the time of induction.

### Example 2. Effects of the supplementation with air/O₂ saturated PFD on the growth rate of high cell density E. coli RB791 pQE30adh cultures (cultivated in EnBasee-Flo MSM- and EnBase^{®}-Flo CM-medium with/without additional ₋glucoamylase)

Example 2 shows that the supplementation with air/O₂ saturated PFD has a positive effect on the growth rate of high cell density *E*. *coli* RB791 pQE30adh cultures, which have been cultivated in a miniaturized fed-batch cultivation system (EnBase^{®}-Flo).

### a) Bacterial strains

The recombinant strain of *Escherichia coli* RB791 pQE30adh expressing the heterologous alcohol dehydrogenase (ADH) was evaluated in the present Example.

### b) Media composition

The media used were the EnBase^{®}-Flo Mineral Salt Medium (MSM) and the EnBase^{®}-Flo Complex Medium (CM) from BioSilta Oy, which differ in their level of organic nitrogen compounds. BioSilta's EnZ I'm solution was used as amyloglucosidase (GAU), wherein 1.5 units of GAU are added 2 hours after inocultation.

### c) Perfluorinated oxygen carrier

Perfluorodecalin (PFD) was evaluated as the liquid perfluorinated oxygen carrier, i.e. unsaturated PFD (PFD₍₊₎), PFD saturated by compressed air (PFDₐᵢᵣ, PFD_{0%}, or PFD saturated by pure oxygen (PFD_{O2}, PFD,_{100%}); see Example 1. PFD solutions with intermediate oxygen concentrations were obtained by mixing of the air or oxygen saturated solutions respectively.
PFD20% = 8ml of PFDₐᵢᵣ + 2ml of PFD₀₂
PFD40% = 6ml of PFDₐᵢᵣ + 4ml of PFD₀₂
PFD60% = 4ml of PFDₐᵢᵣ + 6ml of PFD₀₂
PFD80% = 2ml of PFDₐᵢᵣ + 8ml of PFD₀₂

### d) Analytical methods

The growth of the cells was monitored by spectrophotometrical measurement of the light absorbance of the cell suspension samples collected during culturing, see Example 1.

### e) Cultivation

The E. *coli* RB791 pQE30adh inoculum and the cultivation of the E. *coli* cells were performed according to Example 1. Cells were precultivated for 2 hours before the initial addition of 1.5 unit of amyloglucosidase (GAU). Enzyme should not be added immediately after inoculation in order to prevent the immediate release of glucose subunits from the carbohydrate polymer.

In the first experiment, the MSM- and the CM-medium were immediately supplemented with PFD (see Fig. 3). In the next experiment, the CM-medium was supplemented with PFD only after 15 hours of cultivation. Futhermore, these cultures were additionally supplemeted with glucoamylase (GA) after 15 hours of cultivation to obtain final concentrations of the amylolytic enzyme of 1.5, 3.0, 4.5, 6.0 and 7.5 GAU L⁻¹ (see Fig. 4).

### f) Results and Discussion

Firstly, OD₆₀₀ values were measured after the E. *coli* cultures had been cultivated for 12 hours on MSM- and CM-media, which differ in their level of organic nitrogen compounds (see Fig. 3). In both media, similar values of E. *coli* density were measured for the control cultures and for the miniaturized fed-batch system supplemented with PFD. Significantly higher values of *E*. *coli* density were observed for the samples harvested from the CM-system than harvested from the MSM-culture. However, this was just the effect of a four times higher initial glucoamylase (GA) concentration than used in the MSM-culture. As a conclusion, it seems that the application of a perfluorinated oxygen carrier cannot improve the cell growth when the cells are cultivated in a miniaturized fed-batch process with a low cell density because their growth is limited by the glucose concentration and not by the concentration of oxygen molecules dissolved in the culture medium.

Therefore, the supplementation with a perfluorinated oxygen carrier should not be done at the beginning of the cultivation but only when the bacterial cells grow to a high cell density. Therefore in the following experiments PFD was added only to the cultures after 15 hours of cultivation. As in the previous cultivation, the medium was supplemented with nitrogen compounds only at the beginning of the cultivation. During this experiment, the same amounts of PFD saturated with different levels of oxygen (from air to pure O₂) were added to cultures only after 15 hours. At this moment, high values of OD₆₀₀ (about OD₆₀₀ = 17) were measured for these standard aerated cultures. After further cultivation regularly the OD₆₀₀ is measured and the results presented in Fig. 4.

In all cultures, the E. *coli* cells grew during the initial 15 hours into glucose limitation as it is normally observed during fed-batch cultivation (Panula-Perälä, J. et al., 2008, Microbial Cell Factories, 7: 31; Krause, M. et al., 2009, Microbial Cell Factories, 9:11). Typical non-exponential growth of the cells was also observed after the culture systems had been supplemented with the perfluorinated oxygen carrier PFD. But the duration of the growth period varied with the glucoamylase concentration added to the culture medium simultaneously with the aerated/oxygenated PFD. The growth proceeded for a longer time in the cultures supplemented with the lower amylase concentration (1.5 and 3.0 GAU L⁻¹) than in the cultures supplemented with the higher amount of the enzyme (4.5 and 6.0 GAU L⁻¹). Furthermore, the final OD₆₀₀ values after 48 hours of cultivation, were up to 25 % higher for the cultures which were supplemented with PFD compared to the control cultures without any PFC.

These results show that the aerated/oxygenated PFD can positively influence the final cell density of E. coli cells cultivated in a miniaturized fed-batch cultivation system. However it should be remarked, that in the presented case nitrogen compounds were only added at the beginning of the cultivation. We know, that the final cell densities may be even higher if nitrogen compounds are added also later during the cultivation (Krause, M. et al., 2009, Microbial Cell Factories, 9:11). Such boosting might further increase the final cell density and prodcution of recombinant proteins.

### Example 3. Effects of the supplementation with PFDs on the growth rate of high cell density E. coli RB791 pOEAdh cultures (cultivated in EnBase^{®}-Flo CM medium with an additional glucoamylase and boosting solution)

Example 3 shows that the postive effect of the supplementation with PFDs on the growth of high cell density E. *coli* RB791 pQE30adh cultures, which have been cultivated in the miniaturized fed-batch cultivation system (EnBase^{®}-Flo) is enhanced, if a glucoamylase and a boosting solution have been additionally added to the medium. In this case, as in example 2, the PFD component was not added at the beginning of the cultivation, but only after 15 hours of cultivation.

### a) Bacterial strain

The recombinant strain of *Escherichia coli* RB791 pQE30adh expressing the heterologous alcohol dehydrogenase (ADH) was evaluated in the present Example.

### b) Media composition

The media used was the EnBase^{®}-Flo Complex Medium (CM) from BioSilta Oy, Boosting solution (10x concentrated 12.0 g L⁻¹ tryptone and 24 g L⁻¹ yeast extract), which is a highly concentrated peptides and aminoacids mixture, was also designed by BioSilta Oy. BioSilta's EnZ I'm solution was used as amyloglucosidase (GAU), wherein 1.5 units of GAU are initially added.

### c) Perfluorinated oxygen carrier

Perfluorodecalin (PFD) was evaluated as the liquid perfluorinated oxygen carrier, i.e. PFD saturated by compressed air (PFD_{0%}), PFD saturated by oxygen enriched air (PFD_{20%}, PFD_{40%}, PFD_{60%}, PFD_{80%}) and PFD saturated by pure oxygen (PFD₍₀₂₎). For information on how these solutions were prepared see Example 2.

### d) Analytical methods

The growth of the cells was monitored by spectrophotometrical measurement of the light absorbance of the cell suspension samples collected during culturing, see Example 1.

### e) Cultivation

The E. *coli* RB791 pQE30adh inoculum and the cultivation of the E. *coli* cells were performed according to Example 2.

Initially, the cells were cultivated in standard EnBase^{®} CM medium without any PFD during the first 15 hours. After 15 hours of incubation, glucoamylase, 0.6 ml boosting soultion of nitrogen compounds and 3.0 ml PFDs were simultaneously applied into the culture medium, whereas the final concentrations of the amylolytic enzyme were 1.5, 3.0, 4.5 and 6.0 GAU / dm³ (see Fig. 5).

### g) Results and Discussion

The results of the E. *coli* cell density during the 24 hours experiment are presented in Fig. 5.

In systems with a final concentration of glucoamylase in the range of 1.5-4.5 GAU / dm³, the final OD₆₀₀ was visibly higher for the cultures supplemented with PFD than the final OD₆₀₀ of the control cultures without any PFD. However, the culture system with 6.0 GAU / dm³ was an exception to the above mentioned rule. This is because high concentrations of glucose, which are intensively released from the supplied polysaccharide polymer, may have limited the growth rate of these bacterial cells and, therefore, almost similar values of OD₆₀₀ have been measured for the control and the PFD supplemented cultures. However, the highest OD₆₀₀ value of the cultures supplemented with PFD measured after 24 hours was approximately 60% higher than the OD₆₀₀ values of the control cultures without PFD measured at the same time. Finally, the highest cell density was noted when the E. *coli* cells were cultivated in the presence of PFD saturated by air enriched with 40% oxygen (PFD_{40%}) at 4.5 GAU / dm³. The OD₆₀₀ values measured for the cultures supplemented with PFD saturated by pure oxygen (PFD_{100%}) were higher than those measured for the control cultures without any PFD but visibly lower than the values of the cultures supplemented with PFD_{40%}.

### Example 4. Effects of the supplementation with PFDs on the expression of the recombinant protein of high cell density E. coli RB791 pQE30adh cultures (cultivated in EnBase®-Flo CM medium with an additional glucoamylase and boosting solution)

Example 4 shows the postive effect of the supplementation with PFDs on the expression of the recombinant protein of high cell density E. *coli* RB791 pQE30adh cultures, which have been cultivated in a miniaturized fed-batch cultivation system (EnBase^{®}-Flo), wherein the medium had been additionally supplemented with a glucoamylase and a boosting solution.

### a) Bacterial Strain

The recombinant strain of *Escherichia coli* RB791 pQE30adh expressing the heterologous alcohol dehydrogenase was evaluated in the present Example.

### b) Media composition

The medium used was the EnBase^{®}-Flo Complex Medium (CM) from BioSilta Oy, for enhancing complex components 12.0 g L⁻¹ tryptone and 24.0 g L⁻¹ yeast extract were added. BioSilta's EnZ I'm solution was used as amyloglucosidase (GAU), wherein 1.5 units of GAU are initially added.

### c) Perfluorinated oxygen carrier

Perfluorodecalin (PFD) was evaluated as the liquid oxygen carrier, i.e. PFD saturated by compressed air (PFD_{0%}) or PFD saturated by air enriched with oxygen (PFD_{40%}); for preparation see Example 2.

### d) Analytical methods

The growth of the cells was monitored by spectrophotometrical measurement of the light absorbance of the cell suspension samples collected during culturing, see Example 1.

The soluble and the insoluble fraction of the cellular proteins was analyzed by the SDS-PAGE method according to Example 1.

### e) Cultivation

The E. *coli* RB791 pQE30adh inoculum and the cultivation of the E. *coli* cells were performed according to Example 3.

Initially, cells were cultivated in standard EnBase^{®} CM medium without any PFD during the first 15 hours. After 15 hours, a glucoamylase and 0.6 ml boosting solution of nitrogen compounds and 3.0 ml PFD were simultaneously applied into the culture medium, whereas the final concentrations of the amylolytic enzyme were 1.5, 3.0 and 4.5 GAU / dm³ (see Fig. 6).

### f) Results and Discussion

Results of the SDS-PAGE analysis of the water soluble and insoluble fractions of proteins, which have been isolated from *E. coli* RB791 pQE30adh cells cultured with or without aerated/oxygenated PFD are shown in Fig. 6.

Initially, a high expression of the alcohol dehydrogenase (ADH), which has been encoded by the pQE30adh plasmid, could be observed in all analyzed samples. Furthermore, it could be seen that in all analyzed samples most of the expressed enzyme molecules had the desired soluble conformation (see Fig. 6A and 6B). It was also observed that in the samples, which were harvested 9 hours after the induction by IPTG (see Fig. 6B), was more soluble ADH than in the samples, which were harvested 3 hours after the induction by IPTG (see Fig. 6A). Thus, the longer the time of cultivation, the more recombinant protein could be obtained in the soluble fraction.

In some of the analyzed samples, the level of the expressed protein in the insoluble form was lower (see Fig. 6C, lines 2-3; Fig. 6D, lines 2-3) than of the control samples, which were not supplemented with PFD (see Fig. 6C, line 1; Fig. 6D, line 1). This may be an effect of the enhanced oxygen transport to the culture medium, which is facilitated by the presence of PFD. It was noticed earlier that culturing bacterial cells at oxygen limitation has a negative influence on the expression of recombinant proteins (Salmon, K. et al., 2003, The Journal of Biologial Chemistry, 278: 29837-29855; Overton, T.W., et al., 2006, Biochemical Society Transactions, 34: 104-107). But mentioned publications have compared results of protein expression under aerobic and anaerobic conditions of the culture. Thus, it is impossible to compare mentioned results with the effects of the enhanced protein production, which was studied during our experiment.

### Example 5. Effects of the supplementation with PFDs on the growth rate and on the plasmid concentration in bacterial cells of high cell density E. coli Top 10 cultures in closed eppendorf vials (cultivated in EnBase^{®}-Flo CM medium with an additional glucoamylase and boosting solution)

Example 5 shows the postive effect of the supplementation with PFDs on the growth rate and on the plasmid concentration of high cell density *E*. *coli Top* 10 cultures, which have been cultivated in the miniaturized fed-batch cultivation system (EnBase^{®}-Flo), wherein a glucoamylase and a boosting solution have been additionally added to the medium.

### a) Bacterial strain

E. *coli* OneShot® Top10 *[F- mcrA Δ(mrr-hsdRMS-mcrBC) ϕ80lacZΔM15 ΔlacX74 recA1 araD139 Δ(ara-leu) 7697 galU galK rpsL (StrR) endA1 nupG* λ- ] by Invitrogen containing the Gateway® Entry-vector (pBR322 replication origin, lac-derived CTU promoter, T7 ribosome binding site and His-tag, described in Siurkus et al. 2010, Microbial Cell Factories 9:35). The plasmid vector also contained the gene for human Wnt5a which however was not induced in this example experiments. The plasmid carries a kanamycine resistance gene.

### b) Media composition

The medium used was the EnBase^{®}-Flo Complex Medium (CM) which was enriched with BioSilta's EnBase^{®}-Booster solution. BioSilta's EnZ I'm solution was used as amyloglucosidase (GAU), wherein 1.5, 3.0, 6.0, 10.0, 15.0 and 20.0 units of GAU L⁻¹ were initially added to the parallel cultures of *E.coli* Top10 cells.

### c) Perfluorinated oxygen carrier

Perfluorodecalin (PFD) was evaluated as the liquid oxygen carrier, i.e. PFD saturated by compressed air (PFD_{0%}) or PFD saturated by air enriched with oxygen (PFD_{40%}); for preparation see Example 2.

### d) Analytical methods

The growth of the cells was monitored by spectrophotometrical measurement of the light absorbance of the cell suspension samples collected during culturing. The OD₆₀₀ value of 1 is equivalent to 1.7 g L⁻¹ of wet weight and to 0.39 g L⁻¹ of dry weight of *E*. *coli* biomass.

Plasmid DNA from a standardized (OD₆₀₀ = 10) lysate of *E*. *coli* Top10 cells was prepared according to Invisorb^{®} Spin Plasmid Mini Two protocol (Invitek GmbH; Berlin, Germany; cat. no.: 10101403). The lysis buffers was directly added to the cultures without centrifugation of the cells and without separating them from the medium and PFD_{40%}- PFCs are widely known as chemically inert. Thus, it is believed that the used PFD did not react with the ingredients of the used buffers. The concentration of plasmid DNA was then directly measured with a NanoDrop ND-1000 UV/VIS spectrophotometer (NanoDrop Technologies; USA).

### e) Cultivation

The inoculum was prepared by washing the plasmid containing *E. coli* Top10 cells with 1 ml EnBase^{®}-Flo CM 10x boosted medium from a LB-plate cultivated overnight at 37°C. The following cultivations were performed in closed 1.5 ml Eppendorf tubes which contained 50 µl of medium. Cultures were incubated on an (Eppendorf Thermomixer at 14.000 rpm (5 mm radius) and 37 °C, for up to 24 h.

The PFD created separate phases as it cannot mix with aqueous systems. Thus, the PFD volume, which has been added to the culture medium, did not change the concentration of its ingredients. 50 µl of PFD_{40%} was added to the cultures at the time of inoculation. Half of the cultures were cultivated with a bottom phase of PFD_{40%} and the control cultures were performed without PFD (no PFD).

Glucoamylase was also added to the cultures at the time of inoculation to obtain final concentrations of the amylolytic enzyme of 1.5, 3.0, 6.0, 10.0, 15.0 and 20.0 GAU L⁻¹.

### f) Results and Discussion

Initially, it could be observed that the supplementation of the miniaturized fed-batch cultivation system EnBase^{®}-Flo with the liquid perfluorinated oxygen carrier PFD_{40%} had positive effects on the cell growth of the plasmid containing *E*. *coli* Top10 cells cultivated in high cell density (see Fig. 7A). Furthermore, higher levels of the isolated plasmid were observed in the presence of PFD_{40%} (Figure 7B). Thus, the applied perfluorinated oxygen carrier enhanced final plasmid yield in the miniaturized fed-batch cultivation system compared to control cultures which did not contain PFD.

## Claims

1. A method for increasing the expression of a recombinant protein or the yield of a plasmid produced in an eukaryotic or prokaryotic cell culture cultivated in an enzyme-based fed-batch cultivation system using a cultivation medium which is supplemented with at least one perfluorochemical.

2. The method of claim 1, wherein the perfluorchemical is added either at the time of inoculation or later at the time when the cells are grown to a high cell density.

3. The method of claim 1 or 2 wherein the prokaryotic cells are *E. coli* cells.

4. The method of claims 2 or 3, wherein high cell density is a cell density where the bacterial cells are grown to a density of 7x10⁹ cells/ ml, preferably to above 1x10¹⁰ cells/ml.

5. The method of any of claims 1-3, wherein the perfluorochemical is 1,1,2,2,3,3,4,4a,5,5,6,6,7,7,8,8,8a-octadecafluorodecalin.

6. The method of any of claims 1-5, wherein the perfluorochemical is saturated by air and enriched with 20-80% pure oxygen.

7. The method of claims 1-6, wherein the cultivation medium is supplemented with a further amount of an amylolytic enzyme.

8. The method of claim 6, whererin the final concentration of the amylolytic enzyme is 0.01-100 U L⁻¹ .

9. The method of any of claims 1-8, wherein the cultivation medium is supplemented with a boosting solution containing peptides and amino acids.

10. The method of claim 9 wherein the boosting solution is supplemented with tryptone and yeast extract.

11. The method of claim 9 or 10, wherein the boosting solution contains 10x concentrated 12 g L⁻¹ tryptone and 24 g L⁻¹ yeast extract.

12. The method of any of claims 1-11 wherein the culture is shaken.
